# EUROPÄISCHE PATENTSCHRIFT

(11) **EP 2 490 810 B1**
(45) Veröffentlichungstag und Bekanntmachung des Hinweises auf die Patenterteilung: **07.05.2014**
(21) Anmeldenummer: 10745597.4
(22) Anmeldetag: 20.08.2010
(51) Int. Cl.: B01L 3/00, A61B 10/00, B01F 13/00

(54) **TESTSET FÜR EINE PHOTOMETRISCHE MESSEINRICHTUNG UND PHOTOMETRISCHES MESSVERFAHREN FÜR EINE PROBENFLÜSSIGKEIT**
TEST SET FOR A PHOTOMETRIC MEASURING DEVICE AND PHOTOMETRIC MEASURING METHOD FOR A SAMPLE LIQUID
ENSEMBLE POUR EFFECTUER DES TESTS POUR UN DISPOSITIF DE MESURE PHOTOMÉTRIQUE ET PROCÉDÉ DE MESURE PHOTOMÉTRIQUE D'UN ÉCHANTILLON DE LIQUIDE

(30) Priorität: 22.10.2009 AT 16612009
(43) Veröffentlichungstag der Anmeldung: 29.08.2012
(73) Patentinhaber: Bonecker, Gerhard, 6340 Baar (CH)
(72) Erfinder: Bonecker, Gerhard, 6340 Baar (CH)
(74) Vertreter: Babeluk, Michael
(86) Internationale Anmeldenummer: PCT/EP2010/062163
(87) Internationale Veröffentlichungsnummer: WO 2011/047902

(56) Entgegenhaltungen:
- WO-A1-99/31218
- WO-A1-2005/071388
- WO-A2-2007/053870
- US-A1- 2003 143 752

## Beschreibung

Die Erfindung betrifft ein Testset für eine photometrische Messeinrichtung bestehend aus einem Mischbehälter, der in seinem Inneren eine erste Flüssigkeit enthält und ein von seiner Füllöffnung abnehmbares Verschlusselement aufweist, sowie einem Dosierbehälter, der in einem verschlossenen Hohlraum eine zweite Flüssigkeit enthält, wobei der Dosierbehälter dichtend in die Füllöffnung des Mischbehälters einsetzbar ist, wobei durch eine Druckbeaufschlagung auf einen Verschlusskolben des Dosierbehälters die zweite Flüssigkeit in das Innere des Mischbehälters befördert und mit dieser vermischt werden kann. Weiters betrifft die Erfindung ein photometrisches Messverfahren für eine Probenflüssigkeit, welche mit einer ersten und einer zweiten Flüssigkeit vermischt wird.

Bei vielen medizinischen Tests muss die zu vermessende Probe zunächst mit einer ersten Flüssigkeit in Kontakt gebracht werden, um die Probe zu konditionieren, für die Messung vorzubereiten oder um eine erste chemische oder biologische Reaktion auszulösen. In einem zweiten Schritt wird dann eine zweite Flüssigkeit zugesetzt, um den zu bestimmenden Analyten der Probe in einen für die photometrische Messung geeigneten Zustand zu überführen oder eine zweite chemische oder biologische Reaktion auszulösen. Beispielsweise wird bei einer sogenannten CRP-Messung (C-Reaktives Protein), welche zur Unterscheidung viraler oder bakterieller Entzündungen dient, eine Blutprobe mit einer Lysereagenz vermischt und danach eine Latexreagenz zugesetzt und vermischt, wobei die chemische Reaktion mit Hilfe eines Photometers gemessen wird.

Ein Testset der eingangs genannten Art ist beispielsweise aus der WO 2007/053870 A2 bekannt. Das Testset umfasst einen Mischbehälter, sowie einen in den Mischbehälter einsetzbaren Dosierbehälter. Der Mischbehälter ist mit einem von einer Füllöffnung abnehmbaren Verschlusselement ausgestattet und beinhaltet eine erste Flüssigkeit, wobei der Dosierbehälter nach der Abnahme des Verschlusselementes und der Zugabe der Probenflüssigkeit in die erste Flüssigkeit in die Füllöffnung des Mischbehälters eingesetzt werden kann. Der Dosierbehälter weist in einem verschlossenen Hohlraum eine zweite Flüssigkeit auf, wobei der Hohlraum auf einer Seite durch einen Verschlusskolben und auf der gegenüberliegenden Seite durch einen beweglichen Stopfen verschlossen ist, welcher nach einer Druckbeaufschlagung auf den Verschlusskolben die zweite Flüssigkeit zusammen mit dem beweglichen Stopfen in das Innere des Mischbehälters befördert. Nach der Vermischung der Probe mit der ersten Flüssigkeit und der zweiten Flüssigkeit wird der Mischbehälter in einen photometrischen Analysator eingesetzt, wonach die Probeninhaltsstoffe photometrisch vermessen werden. Die bekannten Verfahren sind fehleranfällig, da exakte Probenmengen zugeführt werden müssen, die für das jeweilige Testset und den jeweiligen medizinischen Test exakt vorgeschrieben sind.

Aus der WO 2005/071388 A1 ist ein Probennahme- und Messelement bekannt geworden, welches aus mehreren zylindrischen Kompartments besteht, die axial verschiebbar ineinander gesteckt sind, wobei deren Innenräume in der Ausgangsstellung durch eine durchstoßbare Membran verschlossen sind. Zwei der Elemente enthalten Reaktionsmittel, in das dritte Element kann mit einem Tupfer eine Probe eingebracht werden. Die Kompartments werden dann durch Druckausübung auf die beiden äußeren Elemente ineinander geschoben, wodurch die Membranen an den Verbindungsstellen aufreißen und gleichzeitig die beiden Reagenzflüssigkeiten mit der Probe vermischt werden. Eine Analyse erfolgt entweder durch optische Inspektion oder durch Einsatz in ein Messgerät.

Die DE 24 41 724 A1 beschreibt eine Analysenpatrone für photospektrometrische Messungen, welche einen ersten Behälter zur Aufnahme einer ersten Flüssigkeit aufweist, wobei der Behälter zunächst durch ein Verschlusselement verschlossen ist. Nach der Abnahme des Verschlusselementes wird in den Behälter die zu analysierende Probe eingegeben und danach ein Behältereinsatz aufgesetzt, welcher in einer Hilfskammer eine Reagenzflüssigkeit aufweist. Die Hilfskammer ist mit einem in der Ausgangsstellung über den Behältereinsatz hinausragenden zylindrischen Stößel versehen, welcher beim Niederdrücken mit Hilfe einer frontseitigen Schneidkante eine Membran der Hilfskammer aufreißt und damit die zweite Flüssigkeit aus der Hilfskammer in den Behälter mit der ersten Flüssigkeit entlässt. Nachdem sich die Flüssigkeiten vollständig gelöst und vermischt haben, wird der Behälter in der für das Analyseverfahren erforderlichen Weise erwärmt und die Probe photometrisch vermessen.

Aufgabe der Erfindung ist es, ein in der Handhabung vereinfachtes photometrisches Messverfahren für eine Probenflüssigkeit vorzuschlagen, wobei ein verbessertes Testset zum Einsatz kommen soll, mit welchem Fehler bei der exakten Probenzumessung weitgehend vermieden werden sollen. Insbesondere soll auf einfache Weise eine genaue Dosierung der Probenflüssigkeit möglich sein.

Diese Aufgabe wird erfindungsgemäß dadurch gelöst, dass der Dosierbehälter eine integrierte Probennahmeeinrichtung mit einer beidseitig offenen Kapillare aufweist, die nach dem Einsetzen des Dosierbehälters in die Füllöffnung des Mischbehälters mit der im Mischbehälter vorliegenden ersten Flüssigkeit in Kontakt steht.

Insbesondere ist erfindungsgemäß vorgesehen, dass die beidseitig offene Kapillare der Probennahmeeinrichtung ein Volumen zwischen 5 µl und 50 µl aufweist.

Durch die Verwendung eines integrierten Probennahmesystems mit einer an beiden Seiten offenen Kapillare (end-to-end capillare) wird das Testset für den Benutzer wesentlich anwendungsfreundlicher. Die Kapillare füllt sich nach Probenkontakt automatisch mit dem durch den inneren Durchmesser und die Länge der Kapillare vorgegebenen Volumen zwischen beispielsweise 5 µl und 25 µl, so dass der Benutzer keine separaten Pipetierschritte durchführen muss. Der Anwender muss mit dem Ende des Kapillarröhrchens lediglich die Oberfläche der Probenflüssigkeit berühren, wobei sich das Röhrchen durch die Kapillarwirkung füllt und exakt das für die jeweilige Probenmessung vorgegebene Probenvolumen aufgesogen wird.

Das erfindungsgemäße Messverfahren, bei welchem die erste Flüssigkeit in einem zunächst verschlossenen Mischbehälter und die zweite Flüssigkeit in einem Dosierbehälter vorliegt, dessen Hohlraum durch einen Stopfen verschlossen ist, zeichnet sich somit durch folgende Schritte aus:
- Aufnehmen der Probenflüssigkeit mittels einer am Dosierbehälter befestigten, beidseitig offenen Kapillare, wobei die Probenflüssigkeit mittels Kapillarwirkung eingesogen wird;
- Öffnen des Mischbehälters;
- Einsetzen des Dosierbehälters in den Mischbehälter, wobei die in der Kapillare vorliegende Probenflüssigkeit in den Mischbehälter eingebracht wird;
- Vermischen der ersten Flüssigkeit mit der Probenflüssigkeit durch Schütteln des Mischbehälters;
- Einbringen der zweiten Flüssigkeit aus dem Dosierbehälter in den Mischbehälter, wobei Druck auf die zweite Flüssigkeit ausgeübt und diese zusammen mit dem Stopfen in die erste Flüssigkeit entlassen wird;
- Vermischen der ersten Flüssigkeit, der Probenflüssigkeit und der zweiten Flüssigkeit;
- Photometrische Messung der chemischen Reaktion in einem Analysator und
- Berechnen der Konzentration zumindest eines Probeninhaltsstoffes.

Die Erfindung wird im Folgenden anhand von Zeichnungen näher erläutert. Es zeigen:
- Fig. 1: den Mischbehälter des erfindungsgemäßen Testsets in einer Schnittdarstellung;
- Fig. 2: den Dosierbehälter des erfindungsgemäßen Testsets in einer Schnittdarstellung;
- Fig. 3: den Dosierbehälter gemäß Fig. 2 eingesetzt in den Mischbehälter in einer ersten Messstellung;
- Fig. 4: den Dosierbehälter gemäß Fig. 2 eingesetzt in den Mischbehälter in einer zweiten Messstellung; sowie
- Fig. 5: eine dreidimensionale Darstellung des Dosierbehälters in einer Schnittdarstellung gemäß Fig. 2.

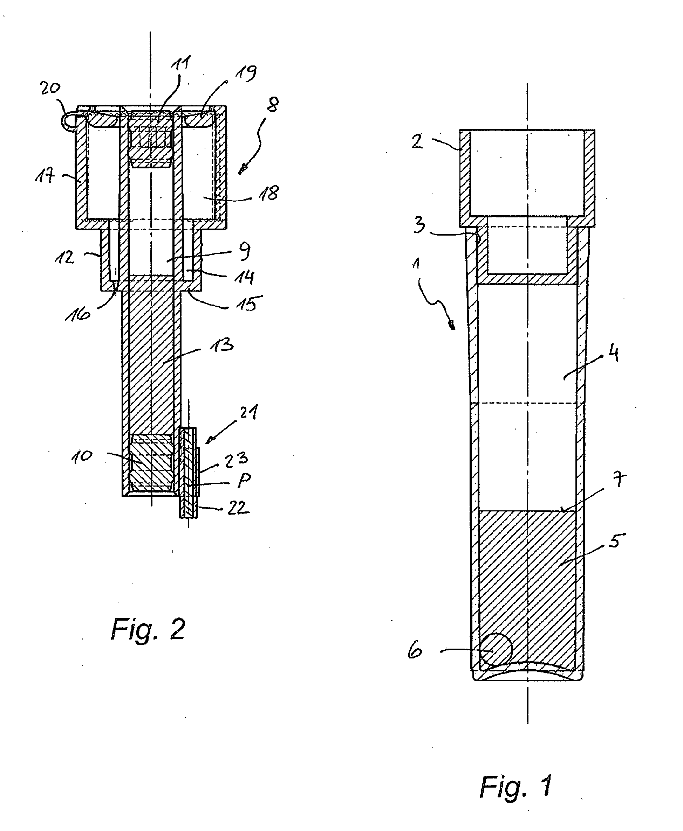

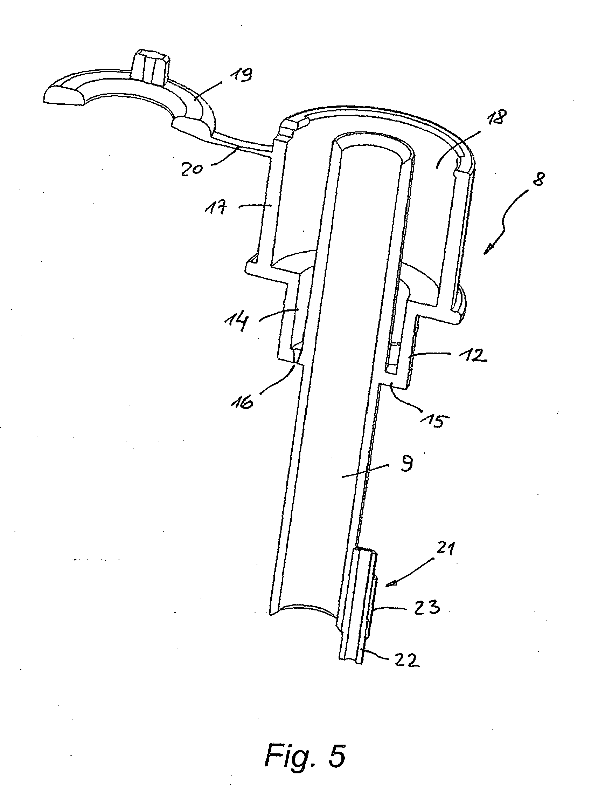

Das in den Fig. 1 bis Fig. 5 dargestellte Testset dient zum Einsatz in eine photometrische Messeinrichtung bzw. einen Analysator, wie beispielsweise in der WO 2007/053380 A2 dargestellt. Der Mischbehälter 1 des Sets weist ein Verschlusselement 2 auf, beispielsweise einen abnehmbaren Kunststoffstoppel, welcher die Füllöffnung 3 verschließt. Im Inneren 4 des Mischbehälters 1 befindet sich eine erste Flüssigkeit 5 sowie ein Magnetrührstab oder eine Stahlkugel 6. Über der ersten Flüssigkeit 5 befindet sich ein Luftraum, wobei die Flüssigkeitsoberfläche mit 7 angedeutet ist.

Der in den Mischbehälter 1 einsetzbare Dosierbehälter 8 weist einen zylindrischen Hohlraum 9 auf, der an einem Ende (austrittseitig) mit einem Stopfen 10 verschlossen ist. An der gegenüberliegenden Seite befindet sich im Hohlraum 9 ein axial verschiebbarer Verschlusskolben 11, welcher entweder manuell nach unten verschoben wird, oder auf welchen ein Betätigungsstempel eines hier nicht dargestellten Analysators Druck ausüben kann.

Die Probennahmeeinrichtung 21 weist eine beidseitig offene Kapillare 22 auf, deren Volumen exakt auf die Anforderungen des jeweiligen Messverfahrens abgestimmt ist und vorzugsweise zwischen 5 µl und 50 µl liegt. Der Benutzer muss die bevorzugt seitlich am Dosierbehälter 8 befestigte und über den Endbereich des Dosierbehälters 8 hinausragende Kapillare 22 lediglich mit der Oberfläche der zu vermessenden Probenflüssigkeit in Kontrakt bringen, wonach die Probenflüssigkeit P automatisch mittels Kapillarwirkung und in der durch das Volumen der Kapillare vorgegebenen Menge in die Probennahmeeinrichtung 21 eingesogen wird. In Fig. 2 ist die mit der Probenflüssigkeit P gefüllte Kapillare 22 dargstellt.

Erfindungsgemäß kann die Kapillare 22 durch Koextrusion hergestellt und am Dosierbehälter 8 angeformt werden. Es kann dabei ein für die Kapillare optimal geeigneter Kunststoff verwendet werden, welcher sich vom Kunststoffmaterial des Dosierbehälters unterscheidet.

Gemäß einer Variante kann die Kapillare 22 in einer am Endbereich des Dosierbehälters 8 angeformten Aufnahme 23 gehalten sein.

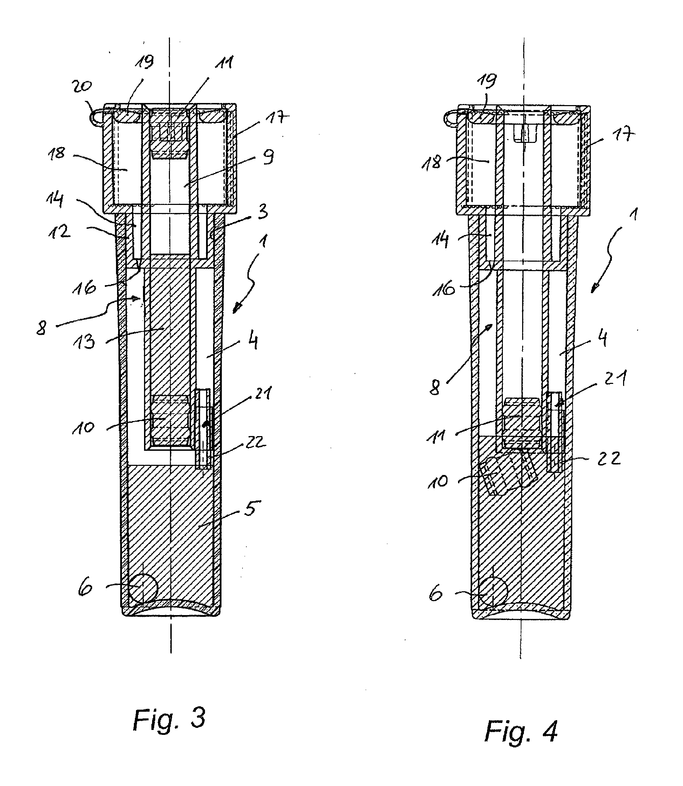

Zum dichtenden Einsatz in den Mischbehälter 1 weist der Dosierbehälter 8 eine zylindrische Dichtfläche 12 auf, die am Dosierbehälter 8 mit einem Ringflansch 14 angeformt ist und einen Ringraum 15 bildet, wobei im Ringflansch 14 zumindest eine Entlüftungsöffnung 16 angeordnet ist. Beim Einsetzen des Dosierbehälters 8 in den Mischbehälter 1 (siehe Fig. 3) kann die vom Dosierbehälter 8 verdrängte Luft aus dem Inneren 4 durch die gasdurchlässige, annähernd tropfendichte Entlüftungsöffnung 16 entweichen. Durch Schütteln des Mischbehälters 1 kann nun die Probenflüssigkeit mit der ersten Flüssigkeit 5 vermischt werden.

Erfindungsgemäß ist anschließend an die zylindrische Dichtfläche 12 des Dosierbehälters 8 ein zylindrisches oder ergonomisch optimiertes Griffelement 17 angeformt, das einen Ringraum 18 begrenzt und nach außen durch ein ringförmiges Spritzschutzelement 19 abschließbar ist. Durch das Spritzschutzelement 19 kann der Austritt von Flüssigkeit aus dem Testset wirksam verhindert werden.

Nach dem Vermischen der ersten Flüssigkeit 5 mit der Probenflüssigkeit P kann im Analysator eine photometrische Kalibriermessung (in der ersten Messstellung des Sets) durchgeführt werden.

Danach wird gemäß Fig. 4 die zweite Flüssigkeit mit Hilfe des Kolbens 11 aus dem Dosierbehälter 8 in das Innere 4 des Mischbehälters 1 transferiert, wobei der Stopfen 10 aus dem Dosierbehälter 8 austritt und im Mischbehälter 1 aufschwimmt. Weiters kann ein auf die Stahlkugel 6 wirkender Magnetrührer des Analysators aktiviert werden, wodurch das Gemisch homogenisiert wird und dann photometrisch vermessen wird (zweite Messstellung des Sets), wie beispielsweise in der WO 2007/053380 A2 dargelegt.

### Erstes Beispiel: INR/PT Test

Die INR-Bestimmung ist ein Test dafür, wie schnell das Blut eines Menschen gerinnt. Der Normalwert von INR ist 1, bei einem INR Wert von z.B. 4 gerinnt das Blut viermal langsamer. Ein hoher INR-Wert bedeutet also, dass die Blutgerinnung nicht so gut funktioniert wie bei gesunden Menschen.

Für die Untersuchung verwendet man Vollblut das direkt vom Patienten abgenommen und in die Kapillare eingebracht wird oder Blutplasma aus mit Zitrat versetzten Abnahmeröhrchen.

Im Mischbehälter 1 befindet sich das erste INR Test Reagenz, im Dosierbehälter 8 das zweite INR Reagenz.

Testablauf des INR Tests:
- Blutprobe wird mit der Kapillare 22 des Dosierbehälters 8 kontaktiert und ein definiertes Probenvolumen eingesogen;
- Mischbehälter 1 ist zunächst mit Verschlusselement 2 verschlossen und mit Lysereagenz (80 µl - 150 µl) gefüllt;
- Verschlusselement 2 wird entfernt, Dosierbehälter (enthält Latexreagenz) wird mitsamt der integrierten Kapillare in den Mischbehälter 1 dichtend eingesetzt;
- Mischbehälter 1 und Dosierbehälter 8 werden in verschlossenem Zustand geschüttelt bis Probenflüssigkeit aus der end-to-end Kapillare 22 in den Mischbehälter 1 ausgetreten ist;
- Mischbehälter 1 und Dosierbehälter 8 werden in eine Messeinrichtung (z.B. Analysator aus WO 2007/053380 A2) eingesetzt;
- Testidentifikation durch die Messeinrichtung (durch RFID-Chip in der Verpackung oder auf dem Mischbehälter);
- Lysereagenz und Probenflüssigkeit werden mit Hilfe eines Magnetrührers der Messeinrichtung vermischt (optional);
- Kalibrierwert wird gemessen (optional)
- Latexreagenz (50 µl - 200 µl) wird mit Hilfe eines Stempels der Messeinrichtung oder manuell durch Druck auf den Verschlusskolben 11 zudosiert;
- Lysereagenz, Probenflüssigkeit und Latexreagenz werden mit Hilfe des Magnetrührers vermischt;
- Chemische Reaktion wird mit Hilfe des Photometers gemessen;
- Gerinnungszeit wird bestimmt.

Der Messbereich der photometrischen Messeinrichtung liegt beispielsweise bei INR 0,5 - INR 5

### Zweites Beispiel: HCY-Test

Chemisch gesehen gehört Homocystein (HCY) zu der Gruppe der sog. Aminosäuren. Im Körper wird Homocystein aus Methionin, einer anderen Aminosäure gebildet, die mit der Nahrung zugeführt wird. Homocystein wird normalerweise schnell wieder abgebaut, wobei Vitamin B6 (Pyridoxin), Vitamin B12 (Cobalamin) und Folsäure benötigt werden.

Homocystein konnte als eigenständiger Risikofaktor für atherosklerotische oder thromboembolische Ereignisse (periphere arterielle Verschlusskrankheit, Schlaganfall, koronare Herzkrankheit (Herzenge, Herzinfarkt), verengende Veränderung der Halsschlagader) identifiziert werden. Bei einer Reihe von weiteren Erkrankungen wie Altersdemenz, Entwicklung von Neuralrohrdefekten (Spina bifida) des Kindes Mutterleib und Blutarmut (Anämie) wurde ein Zusammenhang mit erhöhten Homocysteinspiegeln festgestellt.

Im Mischbehälter 1 befindet sich die erste HCY-Reagenz, im Dosierbehälter 8 die zweite HCY-Reagenz. Der Testablauf erfolgt wie im Beispiel 1.

Zielbereich für Homocystein ist unter 10 µmol/l im Serum.

### Drittes Beispiel: CRP -Test

Als drittes Beispiel wird ein Messablauf eines CRP Tests (C- Reaktives Protein, dient hauptsächlich zur Unterscheidung viraler oder bakterieller Entzündung) dargestellt.

Im Mischbehälter 1 befindet sich als erste Flüssigkeit eine Lysereagenz (1000 µl). Mit der Kapillare 22 des Dosierbehälters 8 werden 5 µl Vollblut aufgesogen. Der Dosierbehälter 8 enthält eine Latexreagenz (250 µl). Zunächst wird die Lysereagenz mit der Vollblutprobe vermischt und ein Kalibrierwert gemessen. Danach wird die Latexreagenz zudosiert und nach der chemischen Reaktion der Konzentrationswert photometrisch bestimmt. Der Testablauf erfolgt wie im Beispiel 1

Der Messbereich der photometrischen Messeinrichtung liegt beispielsweise bei 0,2 mg/dl bis 6 mg/dl.

Als Vorteile des erfindungsgemäßen Testsets sind insbesondere anzuführen:
- Anwender muss keine separaten Pipetierschritte durchführen;
- hohe Genauigkeit des aufgenommen Probenvolumens;
- große Zeitersparnis bei der Probennahme;
- Kostenersparnis durch Wegfall separater Probennahmeeinrichtungen.

## Patentansprüche

1. Testset für eine photometrische Messeinrichtung, mit
- einem Mischbehälter (1), der in seinem Inneren (4) eine erste Flüssigkeit (5) enthält und ein von seine Füllöffnung (3) abnehmbares Verschlusselement aufweist,
- mit einem Dosierbehälter (8), der in einem verschlossenen Hohlraum (9) eine zweite Flüssigkeit (13) enthält, wobei der Hohlraum (9) an einer Seite durch einen verschiebbaren Verschlusskolben (11) und an der gegenüberliegenden Seite durch einen beweglichen Stopfen (10) verschlossen ist, und wobei der Dosierbehälter (8) dichtend in die Füllöffnung (3) des Mischbehälters (1) einsetzbar ist,
**dadurch gekennzeichnet, dass** der Dosierbehälter (8) eine integrierte Probennahmeeinrichtung (21) mit einer beidseitig offenen Kapillare (22) aufweist, die nach dem Einsetzen des Dosierbehälters (8) in die Füllöffnung (3) des Mischbehälters (1) mit der im Mischbehälter (1) vorliegenden ersten Flüssigkeit (5) in Kontakt steht.

2. Testset nach Anspruch 1, **dadurch gekennzeichnet, dass** die beidseitig offene Kapillare (22) der Probennahmeeinrichtung (21) ein Volumen zwischen 5 µl und 50 µl aufweist.

3. Testset nach Anspruch 1 oder 2, **dadurch gekennzeichnet, dass** die Kapillare (22) in einer am Endbereich des Dosierbehälters (8) angeformten Aufnahme (23) gehalten ist.

4. Testset nach Anspruch 1 oder 2, **dadurch gekennzeichnet, dass** die Kapillare (22) durch Koextrusion hergestellt und am Dosierbehälter (8) angeformt ist.

5. Testset nach einem der Ansprüche 1 bis 4, **dadurch gekennzeichnet, dass** die Kapillare (22) seitlich am Dosierbehälter (8) befestigt ist und über den Endbereich des Dosierbehälters (8) hinausragt.

6. Testset nach einem der Ansprüche 1 bis 5, **dadurch gekennzeichnet, dass** der Dosierbehälter (8) eine zylindrische Dichtfläche (12) zum Einsatz in den Mischbehälter (1) aufweist, die am Dosierbehälter (8) mit einem Ringflansch (14) angeformt ist und einen Ringraum (15) bildet, wobei im Ringflansch (14) zumindest eine Entlüftungsöffnung (16) angeordnet ist.

7. Testset nach Anspruch 6, **dadurch gekennzeichnet, dass** anschließend an die zylindrische Dichtfläche (12) ein beispielsweise zylindrisches oder ergometrisch optimiertes Griffelement (17) angeformt ist, das einen Ringraum (18) begrenzt und nach außen durch ein ringförmiges Spritzschutzelement (19) abschließbar ist.

8. Photometrisches Messverfahren für eine Probenflüssigkeit (P) mit einer ersten (5) und eine zweiten Flüssigkeit (13) vermischt wird, wobei die erste Flüssigkeit (5) in einem verschlossenen Mischbehälter (1) und die zweite Flüssigkeit in einem Dosierbehälter (8) vorliegt, dessen Hohlraum (9) durch einen Stopfen (10) verschlossen ist, **gekennzeichnet durch** folgende Schritte:
- Aufnehmen der Probenflüssigkeit (P) mittels einer am Dosierbehälter (8) befestigten, beidseitig offenen Kapillare (22), wobei die Probenflüssigkeit (P) mittels Kapillarwirkung eingesogen wird;
- Öffnen des Mischbehälters (1);
- Einsetzen des Dosierbehälters (8) in den Mischbehälter (1), wobei die in der Kapillare (22) vorliegende Probenflüssigkeit (P) in den Mischbehälter (1) eingebracht wird;
- Vermischen der ersten Flüssigkeit (5) mit der Probenflüssigkeit (P) durch Schütteln des Mischbehälters (1);
- Einbringen der zweiten Flüssigkeit (13) aus dem Dosierbehälter (8) in den Mischbehälter (1), wobei Druck auf die zweite Flüssigkeit (13) ausgeübt und diese zusammen mit dem Stopfen (10) in die erste Flüssigkeit (5) entlassen wird;
- Vermischen der ersten Flüssigkeit (5), der Probenflüssigkeit (P) und der zweiten Flüssigkeit (13);
- Photometrische Messung der chemischen Reaktion in einem Analysator und
- Berechnen der Konzentration zumindest eines Probeninhaltsstoffes.

9. Messerverfahren nach Anspruch 8, **dadurch gekennzeichnet, dass** nach dem Vermischen der ersten Flüssigkeit (5) mit der Probenflüssigkeit (P) eine photometrische Kalibriermessung durchgeführt wird.

10. Messverfahren nach Anspruch 8 oder 9, **dadurch gekennzeichnet, dass** die zweite Flüssigkeit (13) mit Hilfe eines Kolbens (11) aus dem Dosierbehälter (8) in das Innere des Mischbehälters (1) transferiert wird.

## Claims

1. A test set for a photometric measuring device, comprising
- a mixing container (1) which receives a first fluid (5) in its interior (4) and a closing element which can be removed from its filling opening (3);
- a dosing container (8) which contains a second fluid (13) in a sealed hollow chamber (9), with the hollow chamber (9) being sealed on one side by a displaceable sealing plunger (11) and on the opposite side by a movable plug (10), and with the dosing container (8) being insertable in a sealing manner into the filling opening (3) of the mixing container (1);
**characterised in that** the dosing container (8) comprises an integrated sample-taking device (21) with a capillary (22) open on both sides, which after the insertion of the dosing container (8) in the filling opening (3) of the mixing container (1) is in contact with the first fluid (5) present in the mixing container (1).

2. A test set according to claim 1, **characterised in that** the capillary (22) of the sample-taking device (21), which capillary is open on both sides, has a volume of between 5 µl and 50 µl.

3. A test set according to claim 1 or 2, **characterised in that** the capillary (22) is held in a receptacle (23) integrally attached to the end region of the dosing container (8).

4. A test set according to claim 1 or 2, **characterised in that** the capillary (22) is produced by co-extrusion and is integrally attached to the dosing container (8).

5. A test set according to one of the claims 1 to 4, **characterised in that** the capillary (22) is fastened laterally to the dosing container (8) and protrudes beyond the end region of the dosing container (8).

6. A test set according to one of the claims 1 to 5, **characterised in that** the dosing container (8) comprises a cylindrical sealing area (12) for insertion into the mixing container (1), which sealing area is integrally attached to the dosing container (8) with an annular flange (14) and forms an annular space (15), with at least one venting opening (16) being arranged in the annular flange (14).

7. A test set according to claim 6, **characterised in that** a handle element (17) which is cylindrically or ergometrically optimized for example is integrally attached adjacent to the cylindrical sealing area (12), which handle element delimits an annular space (18) and can be sealed to the outside by an annular splash protection element (19).

8. A photometric measuring method for a sample fluid (P) which is mixed with a first fluid (5) and second fluid (13), with the first fluid (5) being present in a sealed mixing container (1) and the second fluid being present in a dosing container (8), the hollow chamber (9) of which is sealed by a plug (10), **characterised by** the following steps:
- taking the sample fluid (P) by means of a capillary (22) which is fixed to the dosing container (8) and is open on both sides, with the sample fluid (P) being sucked in by means of capillary effect;
- opening of the mixing container (1);
- inserting the dosing container (8) into the mixing container (1), with the sample fluid (P) present in the capillary (22) being introduced into the mixing container (1);
- mixing of the first fluid (5) with the sample fluid (P) by shaking the mixing container (1);
- introducing the second fluid (13) from the dosing container (8) into the mixing container (1), with pressure being exerted on the second fluid (13) and with the same being released together with the plug (10) into the first fluid (5);
- mixing of the first fluid (5), the sample fluid (P) and the second fluid (13);
- photometric measurement of the chemical reaction in an analyzer, and
- calculating the concentration of at least one sample ingredient.

9. A measuring method according to claim 8, **characterised in that** a photometric calibration measurement is performed after the mixing of the first fluid (5) with the sample fluid (P).

10. A measuring method according to claim 8 or 9, **characterised in that** the second fluid (13) is transferred by means of a plunger (11) from the dosing container (8) into the interior of the mixing container (1).

## Revendications

1. Ensemble de test pour un dispositif de mesure photométrique comportant :
- un récipient de mélange (1) qui renferme un premier liquide (5) à sa partie interne (4) et comporte un élément de fermeture pouvant être extrait de son ouverture de remplissage (3),
- un récipient doseur (8) qui renferme un second liquide (13) dans une cavité fermée (9), cette cavité fermée (9) étant fermée à une extrémité par un piston de fermeture mobile en translation (11) et à son extrémité opposée par un bouchon mobile (10), le récipient de dosage (8) pouvant être monté de façon étanche dans l'ouverture de remplissage (3) du récipient de mélange (1).
**caractérisé en ce que**
le récipient de dosage (8) comporte un dispositif de prélèvement d'échantillon intégré (21) comportant un capillaire (22) ouvert de part et d'autre, qui, après mise en place du récipient de dosage (8) dans l'ouverture de remplissage (3) du récipient de mélange (1) est en contact avec le premier liquide (5) situé dans le récipient de mélange (1).

2. Ensemble de test conforme à la revendication 1,
**caractérisé en ce que**
le capillaire (22) ouvert de part et d'autre du dispositif de prélèvement d'échantillon (21) présente un volume compris entre 5 µl et 50 µl.

3. Ensemble de test conforme à la revendication 1 et 2,
**caractérisé en ce que**
le capillaire (22) est maintenu dans un logement (23) formé dans la zone d'extrémité du récipient de dosage (8).

4. Ensemble de test conforme à la revendication 1 et 2,
**caractérisé en ce que**
le capillaire (22) est fabriqué par co-extrusion et est formé sur le récipient de dosage (8).

5. Ensemble de test conforme à l'une des revendications 1 à 4,
**caractérisé en ce que**
le capillaire (22) est fixé latéralement sur le récipient de dosage (8) et dépasse de la zone d'extrémité de ce récipient de dosage (8).

6. Ensemble de test conforme à l'une des revendications 1 à 5,
**caractérisé en ce que**
le récipient de dosage (8) comporte une surface d'étanchéité cylindrique (12) destinée à être mise en place dans le récipient de mélange (1), qui est formée sur le récipient de dosage (8) avec une bride annulaire (14) et forme une chambre annulaire (15), au moins une ouverture de dégazage (16) étant située dans la bride annulaire (14).

7. Ensemble de test conforme à la revendication 6,
**caractérisé en ce que**
dans le prolongement de la surface d'étanchéité cylindrique (12) est formé un élément de préhension (17) notamment cylindrique ou de forme optimisée d'un point de vue ergonomique, qui limite une chambre annulaire (18) et peut être obturé vers l'extérieur par un élément de protection anti-projections annulaire (19).

8. Procédé de mesure photométrique d'un liquide échantillon (P) par lequel un premier liquide (5) et un second liquide (13) sont mélangés, le premier liquide (5) étant contenu dans un récipient de mélange fermé (1) tandis que le second liquide est contenu dans un récipient de dosage (8) dont le volume interne (9) est fermé par un bouchon (10),
**caractérisé par**
la mise en oeuvre des étapes suivantes :
- prélèvement du liquide échantillon (P) au moyen d'un capillaire (22) ouvert de part et d'autre fixé sur le récipient de dosage (8), le liquide échantillon (P) étant aspiré par capillarité,
- ouverture du récipient de mélange (1),
- mise en place du récipient de dosage (8) dans le récipient de mélange (1), le liquide échantillon (P) présent dans le capillaire (22) étant transféré dans le récipient de mélange (1),
- mélange du premier liquide (5) avec le liquide échantillon (P) par agitation du récipient de mélange (1),
- transfert du second liquide (13) du récipient de dosage (8) dans le récipient de mélange (1), une pression étant exercée sur le second liquide (13), et celui-ci étant renvoyé avec le bouchon (10) dans le premier liquide (5),
- mélange du premier liquide (5), du liquide échantillon (P) et du second liquide (13),
- mesure photométrique de la réaction chimique dans un analyseur, et
- calcul de la concentration d'au moins un composant de l'échantillon.

9. Procédé de mesure conforme à la revendication 8,
**caractérisé en ce qu'**
après le mélange du premier liquide (5) avec le liquide échantillon (P), une mesure de calibration photométrique est mise en oeuvre.

10. Procédé de mesure conforme à la revendication 8 ou 9,
**caractérisé en ce que**
le second liquide (13) est transféré à l'aide d'un piston (11) du récipient de dosage (8) à la partie interne du récipient de mélange (1).
